# EUROPEAN PATENT SPECIFICATION

(11) **EP 3 755 294 B1**
(45) Date of publication and mention of the grant of the patent: **13.11.2024**
(21) Application number: 19710223.9
(22) Date of filing: 22.02.2019
(51) Int. Cl.: A61F 13/537, C09J 123/08

(54) **SELF-SUPPORTING FIBERIZED WEB INCLUDING A HOT MELT COMPOSITION FOR USE IN DISPOSABLE ABSORBENT ARTICLES AND APPLICATION METHOD**
SELBSTTRAGENDE, FASERIGE BAHN MIT EINER HEISSSCHMELZZUSAMMENSETZUNG ZUR VERWENDUNG IN ABSORBIERENDEN WEGWERFARTIKELN UND ANWENDUNGSVERFAHREN
BANDE FIBRÉE AUTOPORTANTE COMPRENANT UNE COMPOSITION THERMOFUSIBLE DESTINÉE À ÊTRE UTILISÉE DANS DES ARTICLES ABSORBANTS JETABLES ET MÉTHODE D'APPLICATION

(30) Priority: 23.02.2018 US 201862634495 P; 31.07.2018 US 201862712695 P
(43) Date of publication of application: 30.12.2020
(73) Proprietor: H.B. Fuller Company, St. Paul, Minnesota 55164-0683 (US)
(72) Inventor: HARRIS, Michael, W., Shoreview, MN 55126 (US); MISRA, Tara, Shoreview, MN 55126 (US); HANSON, Nolan, T., Minneapolis, MN 55412 (US); MOUA, Pag, St. Paul, MN 55119 (US)
(74) Representative: Maiwald GmbH
(86) International application number: PCT/US2019/019243
(87) International publication number: WO 2019/165273

(56) References cited:
- US-A- 5 681 305
- US-A1- 2012 316 528
- US-A1- 2013 006 205
- US-B1- 6 297 309

## Description

### BACKGROUND

Adhesives are often used to bond substrates together so as to maintain the two substrates in a fixed relation to each other. In the area of industrial adhesives, hot melt adhesives are commonly used to bond together a wide variety of articles including disposable absorbent articles comprising non-woven substrates, e.g., adult incontinence products, disposable diapers, sanitary napkins, bed pads, puppy pads, medical dressings, etc.

There can be multiple hot melt adhesives used in the manufacture of a disposable absorbent article. For example, in the manufacture of a disposable diaper, hot melt adhesives are used in construction (e.g., bonding the back sheet to the nonwoven and optionally the absorbent pad), elastic attachment (e.g., bonding the elastic material to the back sheet in, for example, the leg or waist area), and for core stabilization (e.g., applying an adhesive to the absorbent core to increase the strength of the core).

US 2012/316528 discloses disposable absorbent diapers, wherein the absorbent core of the disposable absorbent diapers includes at least one absorbent structure comprising a substrate layer and an absorbent layer with channels.

US 2013/006205 relates to an absorbent structure comprising absorbent polymer particles supported by a substrate layer and immobilized on this substrate layer by a thermoplastic adhesive material and further comprising a component sorbing oily substances that may migrate from the thermoplastic adhesive material.

US 5,681,305 discloses absorbent products and components for use in absorbent products. These absorbent products comprise pressure-sensitive adhesive microfibers and thermoplastic polymer microfibers.

Hot melt adhesives can also be used to bond sub layers together within the disposable article. In particular, hot melt adhesives can be used to bond the top sheet to the acquisition distribution layer (ADL), also known as the transfer layer.

The acquisition distribution layer is a sub-layer immediately above the core that is designed to improve fluid distribution into the core.

The presence of various layers can complicate production. It would be desirable if the manufacturing process could be simplified.

### SUMMARY

Typical standard ADL's include, e.g., carded nonwovens and apertured films. These materials are supplied to disposable article manufacturers as a rolled good. The placement of the ADL in the disposable absorbent article manufacturing process can be complex as the ADL generally only covers a targeted portion of the core so the rolled good needs to be unwound, cut, registered, and bonded in place.

The inventors have found that a hot melt composition can be utilized to form a self-supporting fiberized web that can function as an Acquisition Distribution Layer (ADL). The self-supporting fiberized web forms an effective ADL as witnessed by Fluid Acquisition Time testing and can be applied in line, simplifying the manufacturing process.

In one aspect, the invention includes a disposable absorbent article including:
a top sheet; an absorbent core; and a self-supporting fiberized web situated between the top sheet and the absorbent core, wherein the self-supporting fiberized comprises a hot melt composition having a viscosity of no greater than 50,000 cP at 178°C, and the self-supporting fiberized web has a basis weight of about 10 to about 200 grams per square meter and is free of absorbent materials.

In one embodiment, the disposable absorbent article further including a standard acquisition distribution layer situated either directly above or directly below the self-supporting fiberized web.

In a different embodiment, the hot melt composition is tack free at room temperature.

In another embodiment, the hot melt composition comprises an ethylene copolymer. In one embodiment, the ethylene copolymer is selected from a group consisting of ethylene olefin copolymers and ethylene vinyl acetate copolymers. In some embodiments, the hot melt composition comprises an ethylene copolymer and a wax having a Ring and Ball Softening Point of from about 95°C to about 140°C. In a different embodiment, the wax is selected from a group consisting of Fischer Tropsch wax and polyethylene wax. In one embodiment, the hot melt composition comprises from about 50 % by weight to about 100 % of a thermoplastic polymer.

In another aspect, the invention features a method of forming a forming a self-supporting fiberized web being free of absorbent materials, the method comprising the steps of obtaining a hot melt composition having a viscosity of no greater than 50,000 cP at 178°C; heating the hot melt composition until molten; spraying the hot melt composition, through an applicator; mixing the hot melt composition with air as it comes through the orifices; and applying the hot melt composition as a self-supporting fiberized web having a basis weight of about 10 to about 200 grams per square meter onto a substrate selected from a group consisting of a top sheet, an absorbent core, a core wrap and a standard acquisition distribution layer, wherein the hot melt composition is cooled such that it forms a layer on top of the substrate rather than penetrating into the substrate as it makes contact with the substrate.

In one embodiment, the applicator comprises from 1 to 30 orifices/2.54 cm (1 to 30 orifices/inch). In another embodiment, the air is cooled. In another embodiment, the air is cooled by a tube air cooler.

The present specification also describes a method of applying a self-supporting fiberized web, the method including: providing a nozzle with a hot melt composition channel and an air channel, wherein at least one of the hot melt composition channel and air channel is insulated; delivering a heated hot melt composition to the hot melt composition channel; delivering air to the air channel; mixing the hot melt composition and the air at a nozzle output; and applying the hot melt composition to a substrate.

In one embodiment, the method further includes expelling the hot melt composition at the nozzle output as a sprayed hot melt composition and delivering the sprayed hot melt composition through a cooled forming chamber and onto the substrate. In another embodiment, the forming chamber is a vacuum chamber.

In another embodiment, the method further includes providing a deckle frame within the forming chamber, the deckle frame defining a delivery boundary of the sprayed hot melt on the substrate. In one embodiment, the deckle frame further defines a density of the sprayed hot melt in predefined areas on the substrate. In yet another embodiment, the method further includes delivering a sprayed hot melt composition from the nozzle output to a top side of a forming chamber, wherein a bottom side of the forming chamber includes an air outlet, wherein the air outlet is connected to a vacuum source to remove air from the forming chamber.

The present specification also describes a method of applying a self-supporting fiberized web, the method including: providing a nozzle with a hot melt composition channel and an air channel; delivering a heated hot melt composition to the hot melt composition channel; delivering air to the air channel; expelling the air at a nozzle output; expelling the hot melt composition at the nozzle output as a sprayed hot melt composition; and delivering the sprayed hot melt composition through a cooled forming chamber and onto a substrate.

In one embodiment, the forming chamber is a vacuum chamber. In another embodiment, the sprayed hot melt composition is delivered to a top side of the forming chamber, wherein a bottom side of the forming chamber includes an air outlet, wherein the air outlet is connected to a suction source to remove air from the forming chamber. In another embodiment, a conveyor is configured to pass through the forming chamber in a lateral direction with respect the top and bottom sides, wherein the suction source is provided below the conveyor. In one embodiment, the conveyor is a mesh conveyor and the suction is provided through the mesh conveyor.

In one embodiment, the method further includes providing a deckle frame within the forming chamber, the deckle frame defining a delivery boundary of the sprayed hot melt on the substrate. In another embodiment, the method further includes delivering the substrate to the forming chamber via a conveyor.

The present specification also describes a method of applying a self-supporting fiberized web, the method including: providing a first bank of nozzles, each adjacently spaced from one another; providing a second bank of nozzles, each adjacently spaced from one another, wherein the second bank of nozzles is positioned adjacent the first bank of nozzles, each nozzle of the first and second banks of nozzles including a hot melt composition channel and an air channel; delivering a heated hot melt composition to the hot melt composition channel of each nozzle; delivering air to the air channel of each nozzle; expelling the air at a nozzle output of each nozzle; expelling the hot melt composition at the nozzle output of each nozzle as a sprayed hot melt composition; and simultaneously delivering the sprayed hot melt composition from each nozzle to a substrate.

In one embodiment at least one of the hot melt composition channel and air channel of each nozzle is insulated. In another embodiment, the method further comprises delivering the sprayed hot melt composition from each nozzle output through a cooled forming chamber and onto the substrate.

In a different embodiment, the forming chamber is a vacuum chamber.
In one embodiment, the method further includes providing a deckle frame within the forming chamber, the deckle frame defining a delivery boundary of the sprayed hot melt on the substrate. In another embodiment, the invention further includes delivering a sprayed hot melt composition from the nozzle output of each nozzle to a top side of a forming chamber, wherein a bottom side of the forming chamber includes an air outlet, wherein the air outlet is connected to a vacuum source to remove air from the forming chamber.

### BRIEF DESCRIPTION OF THE DRAWINGS

The following drawings are illustrative of particular embodiments of the present disclosure and therefore do not limit the scope of the present disclosure. The drawings are not to scale and are intended for use in conjunction with the explanations in the following detailed description. Embodiments of the present disclosure will hereinafter be described in conjunction with the appended drawings, wherein like numerals denote like elements.
FIG. 1 illustrates a schematic view of an application system, according to one embodiment of the present disclosure.
FIG. 2 illustrates an example use of the system of FIG. 1 including a forming chamber.
FIG. 3 illustrates another example use of the system of FIG. 1 including a conveyor.
FIG. 4 illustrates another example use of the system of FIG. 1 for manufacturing a disposable absorbent article.
FIG. 5 illustrates another example use of the system of FIG. 1 using banks of nozzles.

### GLOSSARY

A self-supporting fiberized web is a layer of fibers that can support itself without being attached to a substrate.

### DETAILED DESCRIPTION

### DISPOSABLE ABSORBENT ARTICLE

The invention includes a disposable absorbent article including a top sheet, an absorbent core, and a self-supporting fiberized web situated between the top sheet and the absorbent core, wherein the self-supporting fiberized web comprises a hot melt composition having a viscosity of no greater than 50,000 cP at 178°C, and the self-supporting fiberized web has a basis weight of about 10 to about 200 grams per square meter and is free of absorbent materials.

The self-supporting fiberized web can be attached to the top sheet and/or the core by a variety of means including, e.g., hot melt adhesive, thermal bonding, sonic bonding, etc.

Additional layers (e.g., tissue, nonwoven, standard ADL, etc.) can be present between the self-supporting fiberized web, the core, and the top sheet.

The additional layer can be a standard ADL, positioned either directly above or directly below the self-supporting fiberized web. When a standard ADL is used in combination with a self-supporting fiberized web, inventors envision that a lower basis weight (i.e., less expensive standard ADL) can be used.

### TOP SHEET

The top sheet is liquid-permeable and makes up the body facing layer of the disposable absorbent article.

The top sheet may be constructed from a wide range of suitable materials including apertured films, nonwoven webs of natural fibers (e.g., wood or cotton), or synthetic fibers (e.g., olefin fibers (e.g. polypropylene) or polyester), or a combination of such fibers. The top sheet can be spun, bound, or carded. The top sheet can be hydrophobic or hydrophilic (e.g., surfactant treated). The top sheet can be hydrophobic, with the exception of the area in the center, which can be treated with surfactant to be hydrophilic.

### SELF-SUPPORTING FIBERIZED WEB

The invention includes a self-supporting fiberized web that is situated between the top sheet and the absorbent core and can function as an Acquisition Distribution Layer (ADL) or transfer layer, improving the speed at which fluid moves through the top sheet and into the core.

The self-supporting fiberized web is not part of the core but rather assists in transporting fluid into the core. As such, the self-supporting fiberized web is free of absorbent materials such as, e.g., hydrophilic fibers (e.g., cellulose fibers), fluff pulp, super absorbent polymer particles, etc.

The self-supporting fiberized web is formed from a hot melt composition. The self-supporting fiberized web can be applied to the absorbent core facing side of the top sheet or to the top sheet facing side of the core. If the core includes a core wrap, the self-supporting fiberized web can be applied to the core wrap. The self-supporting fiberized web can also be applied to layers immediately above the core, facing the top sheet, e.g., tissue layers, nonwoven layers, standard ADL layers, etc.

The self-supporting fiberized web has a basis weight of from about 10 grams per square meter (gsm) to about 200 gsm, from about 10 gsm to about 100 gsm, or even from about 30 gsm to about 90 gsm.

The self-supporting fiberized web can be tack free at room temperature. Alternatively, the self-supporting fiberized web can have tack at room temperature. When the self-supporting fiberized web has tack, it is anticipated that in addition to helping improve the speed at which fluid moves through the top sheet and into the core, the self-supporting fiberized web could also bond to the core to help improve the strength of the core.

The self-supporting fiberized web can be present as one layer or multiple layers. When present as multiple layers, the layers can comprise different hot melt compositions or can comprise the same hot melt composition. The layers can have different basis weights or the same basis weights. One of the layers can have tack while other layers do not have tack. The tacky layer can assist in attaching the non-tacky layer to other layers of the disposable article (e.g., a portion of core, the top sheet, etc.).

The self-supporting fiberized web is formed by heating the hot melt composition in a heated tank and spraying the hot melt composition with an applicator through a nozzle. The hot melt composition is mixed with air as it comes through the nozzle in order to form fibers. In one embodiment, the air is unheated. In another embodiment, the air is cooled, e.g., by using a tube air cooler e.g. a vortex tube air cooler available from Vortec/ITW Air Management (Cincinnati, Ohio). According to the invention, the hot melt composition is cooled such that it forms a layer on top of the substrate rather than penetrating into the substrate as it makes contact with the substrate.

The design and placement of the self-supporting fiberized web is not limited in any way. The hot melt composition can be applied intermittently so as to only put down a self-supporting fiberized web in a discrete area. This discrete area can be rectangular, or any other shape. The self-supporting fiberized web can be applied with one or more applicator to form layers.

The applicator can be a metered applicator or a pressure fed applicator (e.g., Universal Applicator available from Nordson). The applicator preferably has multiple orifices. The applicator can have from 1 to 30 orifices/inch, from 10 to 30 orifices/inch, from 1 to 15 orifices/inch, or even from 2 to 12 orifices/inch. The orifices can have any possible configuration including side by side, staggered, or any other configuration.

### HOT MELT COMPOSITION

The hot melt composition includes a thermoplastic polymer and optionally a wax or plasticizer. The hot melt composition can include a polymer and a wax.

The hot melt composition can have a Brookfield Viscosity at 178°C (350°F) of from about 100 cP to about 50,000 cP, from about 100 cP to about 30,000 cP, from about 500 cP to about 20,000 cP, or even from about 1000 cP to about 10,000 cP.

### THERMOPLASTIC POLYMER

The hot melt composition includes a polymer. The polymer is present in the hot melt composition at from about 30 % to about 100 %, from about 50 % to about 100 %, from about 40 % to about 95 %, or even from about 50 % to about 90 % by weight.

The polymer can be selected from the group consisting of ethylene homopolymers, ethylene copolymers, propylene copolymers, propylene homopolymers, styrene block copolymers, functionalized versions thereof (e.g., OH modified or maleic anhydride modified), and blends thereof. The polymer can be formed by use of a single-site (e.g., metallocene) catalyst.

If the polymer is a metallocene catalyzed propylene copolymer or homopolymer, it can be blended with a second polymer to optimize melting speed and speed of set.

The polymer can have a melt flow rate according to ASTM 1238D (190°C, 2.16 kg) of from about 100 to about 3000, or even from about 200 to about 2000.

The polymer can be an ethylene copolymer. The term "ethylene copolymer," as used herein, refers to copolymers and terpolymers of ethylene.

Examples of useful ethylene copolymers include ethylene based olefin polymers. Ethylene based olefin polymers include those derived from at least 50 % by weight ethylene and at least one alpha-olefin monomer having at least 3 carbons, or even from 3 to 20 carbon atoms. Useful alpha-olefin monomers include, e.g., propylene, isobutylene, butene, pentene, hexane, heptane, octene, nonene, decene, dodecene, 4-methyl-1-pentene, 3-methyl-1-pentene, 3-methyl pentene-1, 1,3,5,5-trimethyl-hexene-1, 5-ethyl-1-nonene, and combinations thereof. Specific examples of suitable ethylene based olefin polymers include ethylene-propylene, ethylene-hexene, ethylene-octene, and combinations thereof. Useful ethylene based olefin polymers can be prepared using a variety of catalysts including, e.g., a single site catalyst (e.g., metallocene catalysts), multiple single site catalysts, non-metallocene heteroaryl catalysts, and combinations thereof. Useful commercially available ethylene copolymers include AFFINITY GA 1900, AFFINITY GA 1950, AFFINITY GA 1900 H and AFFINITY GA 1875 all available from Dow Chemical Company (Midland, Michigan).

Additional useful ethylene copolymers include copolymers of ethylene and vinyl monomers including, e.g., ethylene vinyl acetate, ethylene methyl acrylate, ethylene ethyl acrylate, ethylene n-butyl acrylate, ethylene acrylic acid, ethylene methacrylate, ethylene methylmethacrylate, ethylene 2-ethylhexyl acrylate, and combinations thereof. Suitable ethylene vinyl acetate copolymers also include a vinyl acetate content of from about 5 % by weight to about 50 % by weight, or even from about 15 % by weight to about 40% by weight. Suitable copolymers of ethylene vinyl acetate are commercially available under the ATEVA series of trade designations including ATEVA 1850A, 1880A, 2830A and 2850A from Celanese Corporation (Dallas, Texas), and the ESCORENE series of trade designations including, e.g., UL8705 from ExxonMobil Chemical Company (Houston, Texas).

### WAX

The hot melt composition can be free of a wax; however, in some preferred embodiments, the hot melt composition includes a wax. The hot melt composition can include from 0 % by weight to about 60 % by weight, from about 5 % by weight to about 50 % by weight, or even from 10 % by weight to about 45 % by weight of wax.

Useful classes of wax include, e.g., paraffin waxes; microcrystalline waxes; high density low molecular weight polyethylene waxes; by-product polyethylene waxes; polypropylene waxes; Fischer-Tropsch waxes; oxidized Fischer-Tropsch waxes; functionalized waxes such as acid, anhydride, and hydroxyl modified waxes; animal waxes; vegetable waxes (e.g., soy wax); and combinations thereof.

The wax can have a Ring and Ball Softening point of from about 95°C to about 140°C, from about 100°C to about 135°C, or even from about 105°C to about 130°C.

The wax can be selected from the group consisting of Fischer-Tropsch waxes and polyethylene waxes.

Useful waxes are commercially available from a variety of suppliers including polyethylene waxes available under the EPOLENE C series of trade designations from Westlake Chemical Corporation (Houston, Texas) and SARAWAX SX 105 available from Evonik Corporation (Parsippany, NJ).

### PLASTICIZER

The hot melt composition can include a plasticizer.

Suitable plasticizers include, e.g., naphthenic oils, paraffinic oils (e.g., cycloparaffin oils), mineral oils, phthalate esters, adipate esters, olefin oligomers (e.g., oligomers of polypropylene, polybutene, and hydrogenated polyisoprene), polybutenes, polyisoprene, hydrogenated polyisoprene, polybutadiene, benzoate esters, animal oil, plant oils (e.g., castor oil, soybean oil), derivatives of oils, glycerol esters of fatty acids, polyesters, polyethers, lactic acid derivatives, and combinations thereof.

Useful commercially available plasticizers include CALSOL 550, napthenic oil from Calumet Specialty Products Partners, LP (Indianapolis, Indiana), KAYDOL OIL, mineral oil from Sonneborn (Tarrytown, New York) PARAPOL polybutene from Exxon Mobil Chemical Company (Houston, Texas), OPPANOL polyisobutylene from BASF (Ludwigsjhafen, Germany), KRYSTOL 550 mineral oil from Petrochem Carless Limited (Surrey, England) and PURETOL 35 mineral oil from Petro Canada Lubricants Inc. (Mississauga, Ontario).

### TACKIFYING AGENT

The hot melt composition can be free of tackifying agent; alternatively, the hot melt composition can include one or more tackifying agents. The hot melt composition can include no greater than about 50 % by weight, no greater than about 20 % by weight, no greater than about 10 % by weight, from 0 % by weight to about 50 % by weight, from 0 % by weight to about 20 % by weight, from about 0 % by weight to about 15 % by weight, from about 5 % to about 40 %, or even from about 2 % to about 10 % by weight of a tackifying agent.

Suitable classes of tackifying agents include, e.g., aromatic, aliphatic and cycloaliphatic hydrocarbon resins, mixed aromatic and aliphatic modified hydrocarbon resins, aromatic modified aliphatic hydrocarbon resins, pure monomer aromatic tackifying resins (e.g., alpha methyl styrene resins), and hydrogenated versions thereof; terpenes, modified terpenes and hydrogenated versions thereof; natural rosins, modified rosins, rosin esters, and hydrogenated versions thereof; low molecular weight polylactic acid; and combinations thereof. Examples of useful natural and modified rosins include gum rosin, wood rosin, tall oil rosin, distilled rosin, hydrogenated rosin, dimerized rosin, and polymerized rosin. Examples of useful rosin esters include, e.g., glycerol esters of pale wood rosin, glycerol esters of hydrogenated rosin, glycerol esters of polymerized rosin, pentaerythritol esters of natural and modified rosins including pentaerythritol esters of pale wood rosin, pentaerythritol esters of hydrogenated rosin, pentaerythritol esters of tall oil rosin, and phenolic-modified pentaerythritol esters of rosin.

Useful tackifying agents are commercially available under a variety of trade designations including, e.g., hydrocarbon tackifying resins under the ESCOREZ series of trade designations, from Exxon Mobil Chemical Company (Houston, Texas) including ESCOREZ 5400, ESCOREZ 5415, ESCOREZ 5600, and pure monomer aromatic tackifying resins under the KRISTALEX and PLASTOLYN series of trade designations from Eastman Chemical Company (Kingsport, Tennessee) including, e.g., PICCOTAC 8095, KRISTALEX 3100, PLASTOLYN 240 and PLASTOLYN 290.

### ADDITIONAL COMPONENTS

The hot melt composition optionally includes additional components including, e.g., stabilizers, antioxidants, additional polymers, adhesion promoters, ultraviolet light stabilizers, corrosion inhibitors, colorants (e.g., pigments and dyes), fragrances, fillers, surfactants, other materials to increase the hydrophilic properties of the hot melt composition (e.g. glycerol monostearate), wetness indicators, and combinations thereof.

Useful antioxidants include, e.g., pentaerythritol tetrakis[3,(3,5-di-tert-butyl-4-hydroxyphenyl)propionate]; 2,2'-methylene bis(4-methyl-6-tert-butylphenol); phosphites including, e.g., tris-(p-nonylphenyl)-phosphite (TNPP) and bis(2,4-di-tert-butylphenyl)4,4'-diphenylene-diphosphonite; di-stearyl-3,3'-thiodipropionate (DSTDP); and combinations thereof. Useful antioxidants are commercially available under a variety of trade designations including, e.g., the IRGANOX series of trade designations including, e.g., IRGANOX 1010, IRGANOX 565, and IRGANOX 1076 hindered phenolic antioxidants and IRGAFOS 168 phosphite antioxidant, all of which are available from BASF Corporation (Florham Park, New Jersey), and ETHYL 702 4,4'-methylene bis (2,6-di-tert-butylphenol). When present, the composition preferably includes from about 0.1% by weight to about 2% by weight antioxidant.

### ABSORBENT CORE

The absorbent core includes the core and optionally a core wrap. The core can vary in design, shape, and materials used. It can be shaped like a long rectangle or like a dog bone. The core is generally centered within the article, and is firmly secured between the top sheet and back sheet. The core includes absorbent materials able to absorb bodily fluids and solids received through the top sheet, the self-supporting fiberized web, and optionally the core wrap. Absorbent materials can include fluff (e.g., cellulose based), super absorbent (e.g., super absorbent particles), nonwoven, tissue, foam, or any other absorbent material.

The core wrap can be any material that wraps around the core to hold the components of the core in place. The core wrap generally wraps around the two longer sides of the core, leaving the ends open. The core wrap can be selected from the group consisting of nonwoven and tissue.

### ADDITIONAL LAYERS

Additional layers (e.g., tissue, nonwoven, etc.) can be present between the self-supporting fiberized web, the core, and the top sheet.

The additional layer can be a standard acquisition distribution layer, situated either directly above or directly beneath the self-supporting fiberized web.

When a standard ADL is included, it can be a hydrophilic nonwoven. A hydrophilic nonwoven has an affinity to water. This affinity can be achieved by using a nonwoven including hydrophilic fibers. Alternatively, a hydrophobic nonwoven can be treated with a chemical (e.g., surfactant, wetting agent, etc.) to improve its affinity to water. The ADL can be a high loft non-woven. When a standard ADL is included, it can be a lower basis weight material - in this manner, the use of the self-supporting fiberize web in combination with the standard ADL can lower the total cost of the disposable absorbent article.

The standard ADL can have a basis weight of from about 10 grams per square meter (gsm) to about 200 gsm, from about 10 gsm to about 100 gsm, from about 30 gsm to about 90 gsm, from about 5 gsm to about 40 gsm, or even from about 10 gsm to about 30 gsm.

### METHODS OF APPLYING THE COMPOSITION DISCLOSED HEREIN

The hot melt composition disclosed herein can be applied in a variety of different ways using a variety of different types of equipment and methods. In some examples, certain equipment used in particular methods of application discussed below can be used in in a variety of different methods of application. In some examples, the hot melt composition disclosed herein can be applied in conjunction with another composition and/or material. In some examples, each composition can be applied intermittently or simultaneously to form separate layers on a substrate.

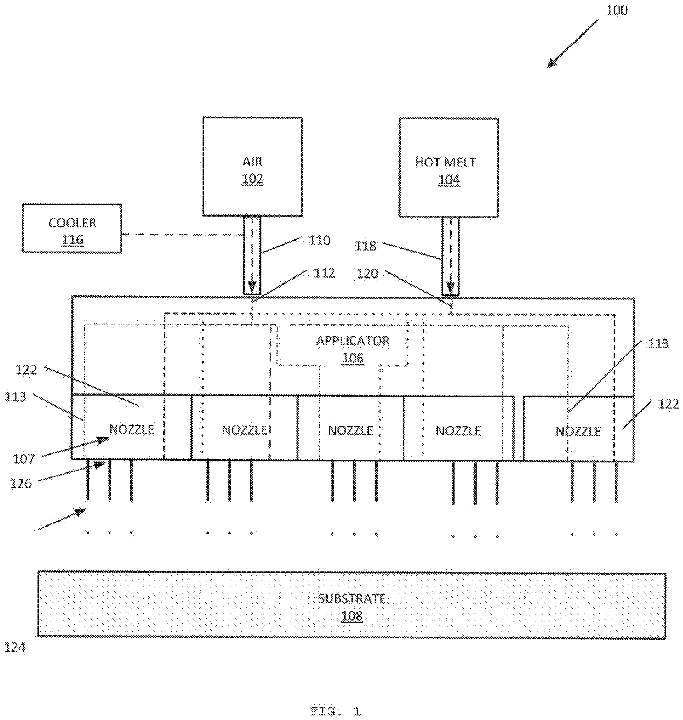

FIG. 1 shows a hot melt composition application system 100. The system 100 includes an air source 102, a hot melt composition source 104, an applicator 106, and a substrate 108. The applicator 106 is shown to include a plurality of nozzles 107. The system 100 shown can be a portion of an overall system. For example, the substrate 108 can be transported past the applicator 106 by conveying means, such as a conveyor. In other examples, a plurality of applicators 106 are used in conjunction with a single substrate 108.

The air source 102 can be any of a variety of air sources such as compressed air, blown air, etc. In some examples, an air source line 110 that connects the air source 102 to the applicator 106 is insulated. In some examples, the applicator 106 has an air inlet 112 and each individual nozzle 107 includes an air channel 113. The air can be heated, unheated or cooled. In a preferred embodiment, the air is unheated or cooled.

In some examples, the air inlet 112 and/or each nozzle air channel 113 are insulated to mitigate heat from any heated hot melt composition within the applicator 106 and nozzles 107 from warming up the air. In some examples, the insulation can be used to prevent the air from being warmed down if the air source is cooled by a cooler 116, such as the vortex cooler, as described above. In some examples, the air delivered to the applicator 106 can be cooled to a temperature between about -1.1°C and 21.1°C (30°F and 70°F). In some examples, the air delivered to the applicator 106 can be cooled to a temperature of between about 12.8°C and 18.3°C (55°F and 65°F).

The hot melt composition source 104 can be a tank, or the like. The hot melt composition from the hot melt composition source 104 is heated and molten. The hot melt composition source 104 can be any of the example ADL hot melt compositions disclosed above. In some examples, the hot melt composition is pumped from the hot melt composition source 104 to the applicator 106. In some examples, a hot melt composition source line 118 to the applicator 106 is insulated. In some examples, like the air source 102 and air channels 113, the applicator 106 has a hot melt composition inlet 120 and each nozzle 107 has a hot melt composition channel 122. In some examples, the hot melt composition inlet 120 and/or each nozzle hot melt composition channel 122 are insulated/shielded to mitigate heat from escaping and from warming up the air in the air channels 113. In some examples, the hot melt composition inlet 120 and/or each nozzle hot melt composition channel 122 are separated a maximum distance from the air inlet 112 and/or each nozzle air channel 113 in the applicator 106 and/or nozzle 107 so as to mitigate heat transfer therebetween.

The applicator 106 can be substantially similar to the applicator disclosed above. Each nozzle 107 of the applicator is configured to be in fluid communication with the air source 102 and the hot melt composition source 104 through the air channel 113 and the hot melt composition channel 122, respectively. In some examples, the nozzles 107 are configured to spray a pattern 124 of hot melt composition out of nozzle outlets 126 onto the substrate 108 that can function as an ADL layer. In some examples, the nozzles may utilize a shim plate stack for air and hot melt composition delivery.

The substrate 108 can be any of a variety of substrates. In some examples, the substrate 108 is the absorbent core. In other examples, the substrate 108 is the top sheet. In still other examples, the substrate 108 is a layer above the core facing the top sheet.

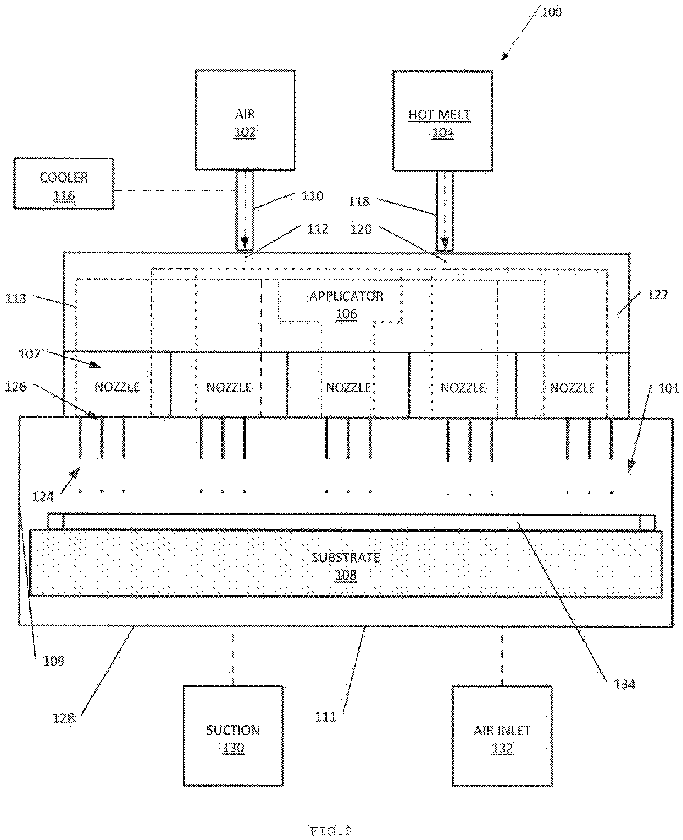

FIG. 2 shows the system 100 including a forming chamber 128. The forming chamber 128 can include an interior volume 101 that the hot melt composition passes through prior to reaching the substrate 108. In some examples, the forming chamber 128 is a box with sides 109. However, the forming chamber 128 can be manufactured from a variety of types of materials in a variety of different shapes. In some examples, the forming chamber 128 can be a box with sides 109 that measure between 80 mm to 100 mm tall and 80 mm to 100 mm wide.

In some examples, the forming chamber 128 cools the hot melt composition from the nozzle outlets 126 prior to the hot melt composition encountering the substrate 108. In other examples, the forming chamber 128 pulls the hot melt composition from the nozzle outlets 126 and onto the substrate 108. The forming chamber 128 can be configured to allow the substrate 108 to pass therethrough so that a plurality of substrates can be treated with hot melt composition without moving the applicator 106 and the forming chamber 128. In some examples, the substrates 108 are delivered to a side 109 of the forming chamber via a conveyor and removed from an opposite side 109 of the forming chamber 128 via the conveyor. In some examples, the substrates 108 are moved in a direction generally transverse to the direction that the hot melt composition leaves the nozzle outputs 126.

In the depicted example, the forming chamber 128 includes a suction source 130 in communication therewith. In some examples, the suction source 130 removes air from the forming chamber 128. In some examples, the suction source 130 creates a vacuum in the forming chamber 128. In other examples, the suction source 130 creates a downward flow of air toward the substrate 108 within the forming chamber 128. In some examples, the forming chamber 128 may also include an air inlet source 132 to provide cooled air to the forming chamber 128. In some examples, the air inlet source 132 provides cooled air at a temperature between -1.1°C and 21.1°C (30°F and 70°F) to the forming chamber 128. As hot melt composition is sprayed from the nozzle outlet 126, the hot melt composition passes through the forming chamber 128 prior to encountering the substrate 108. In some examples, the suction source 130 is positioned at a bottom side 111 of the forming chamber, opposite the applicator 106 and nozzles 107.

In some examples, the forming chamber 128 can include a deckle frame 134 positioned so as to define a delivery boundary of the hot melt composition on the substrate 108. In some examples, the deckle frame 134 can be secured to sides 109 and/or the bottom 111 of the forming chamber 128. In some examples, the deckle frame 134 can be secured to an underside of a mesh conveyor 136. In some examples, the deckle frame 134 can include at least one deckle plate, having of perforations therein. In some examples, the deckle frame 134 can include plurality of deckle plates, some of which have perforations therein. In some examples, the perforations in the plates can alter the amount of suction from suction source 130 that is introduced to the hot melt composition as it leaves the nozzle outlets 126. By altering the suction, higher quantities of the hot melt composition is pulled to certain areas of the substrate 108 than others. These higher quantities can alter the density (i.e., gsm amount) of the hot melt composition delivered to predefined areas of the substrate 108 depending on the positioning of the deckle frame 134 and/or deckle plates. In some examples, the deckle frame 134 can facilitate contouring the hot melt composition at different portions of the substrate 108, for example, delivering hot melt composition at different gsm values to different portions of the substrate 108. In some examples, the deckle frame 134 can allow for hot melt composition to reach 80 gsm in the center of the substrate 108.

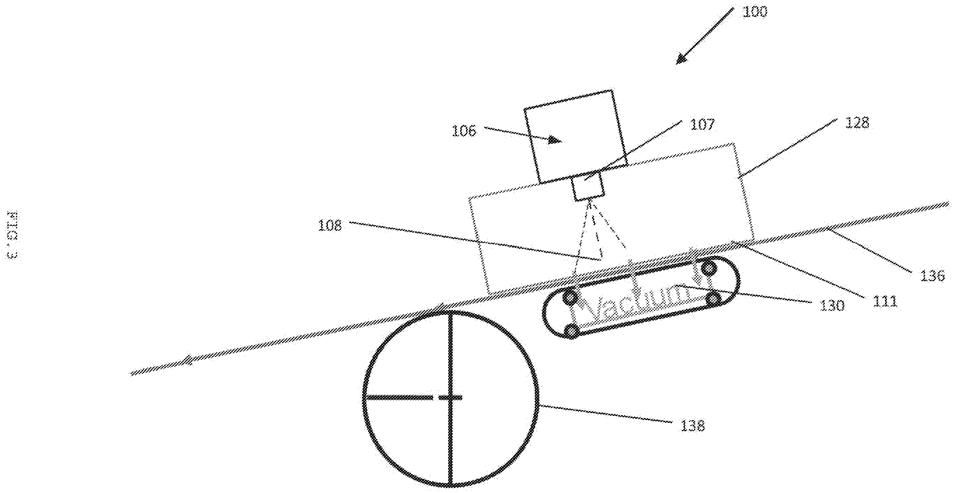

FIG. 3 depicts an example of the system 100 used with a conveyor 136. As shown, the applicator 106 delivers hot melt composition from nozzles 107 to the substrate 108 within the forming chamber 128. The suction/vacuum source 130 is provided at the bottom side 111 of the forming chamber. In the depicted examples, the substrate 108 is delivered to the forming chamber 128 via the conveyor 136 that is moved via rotation means 138. In some examples, the conveyor 136 is a mesh conveyor. In some examples, a vacuum roller/drum can be utilized in place or, or in conjunction with, the conveyor 136, to transport the substrate 108 to the forming chamber 128. In some examples, the suction source 130 is positioned below the conveyor 136 so as to provide a suction through the substrate 108 to pull the hot melt composition onto the substrate 108.

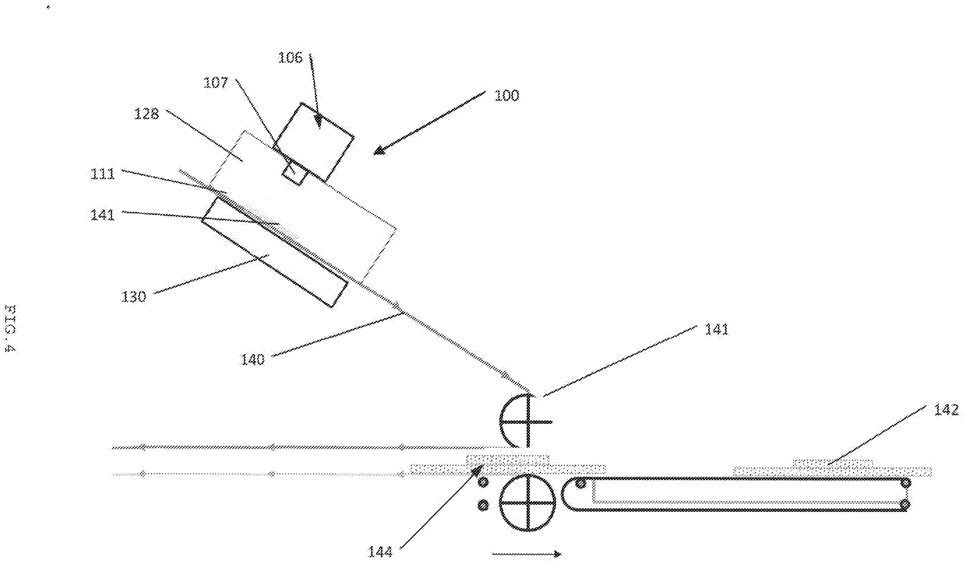

FIG. 4 depicts another of example of the system 100 in use. In the depicted example, the substrate 108 is a top sheet 140 and the hot melt composition is intermittingly applied to the top sheet to form a molten high loft layer 141. As shown, the hot melt composition is applied through the forming chamber 128 and onto the top sheet 140. The substrate is then conveyed so as to join with a core layer 142. The core layer 142 is individually conveyed toward the top sheet with the hot melt composition applied thereto so that the top sheet, hot melt composition, and core join to form a disposable absorbent article 144.

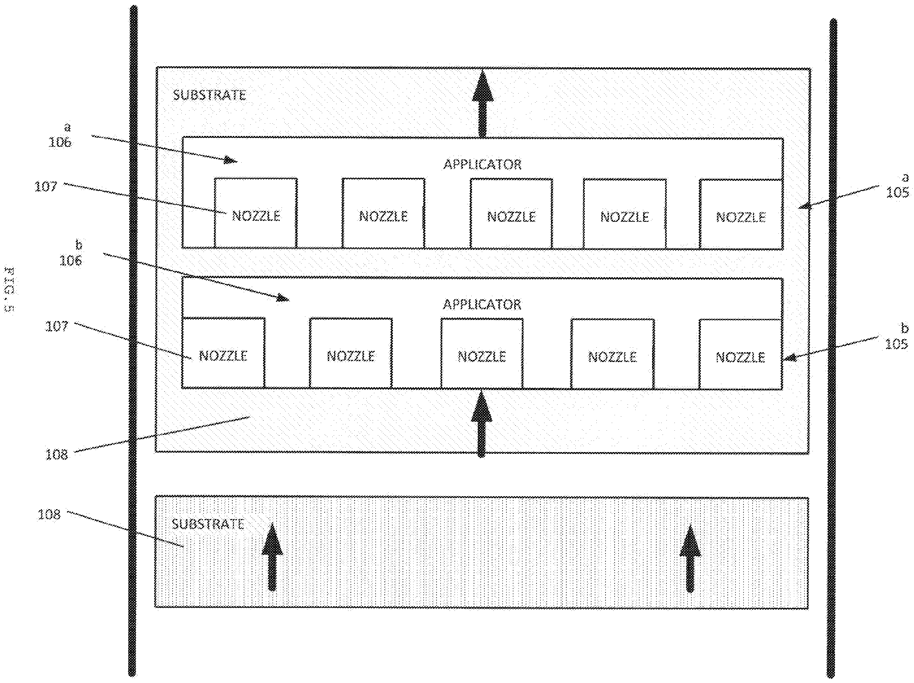

FIG. 5 shows another example of the system 100 in use. As shown, a pair of adjacent applicators 106a, 106b are shown that each include a bank 105a, 105b of nozzles 107 where each nozzle 107 is adjacent one another. As the substrate 108 is moved past the nozzles 107 of the applicators 106a, 106b, the applicators 106a, 106b are configured to deliver hot melt composition to the substrate 108 simultaneously. A variety of different numbers and banks 105 of nozzles 107 can be utilized to form a particular pattern/density of hot melt composition on the substrate 108. In some examples, the nozzles 107 of each applicator 106a, 106b can be aligned with one another in the direction of travel of the substrate 108. In other examples, the nozzles 107 of one applicator 106a can be staggered with the nozzles 107 of the additional applicator106b in the direction of travel of the substrate 108, as shown in FIG. 5.

The invention will now be described by way of the following non-limiting examples. All parts, ratios, percents, and amounts stated in the Examples are by weight unless otherwise specified.

### EXAMPLES

### TEST PROCEDURES

Test procedures are used in the examples and throughout the specification, unless stated otherwise, include the following.

### SAMPLE PREPARATION

The hot melt compositions were prepared in a sigma blade mixer with a batch size of 5000 grams. A heating oil temperature of around 177°C was used. The polymer, tackifying agent and additives were added first, blended until smooth, and then the plasticizer was added in portions. The mixture was allowed to mix until homogeneous and then dumped. Each sample took about one hour to make.

### VISCOSITY TEST METHOD

Viscosity is determined in accordance with ASTM D-3236 entitled, "Standard Test Method for Apparent viscosity of Adhesives and Coating Materials," (October 31, 1988), using a Brookfield Thermosel viscometer Model RVDV 2 and a number 27 spindle. The results are reported in centipoise (cP).

Ring and Ball Softening Point is measured using ASTM D36.

### SPRAY TEST METHOD

A NORDSON UA spray applicator was used equipped with a Signature 12 orifice nozzle. Each composition was sprayed on a standard nonwoven (15 gsm spun bond polypropylene). The following test conditions were used - line speed: 4.57 meters/minute (15 feet/minute); applicator distance from web: 27.9 centimeters (11 inches); air pressure: 103.4 Kilopascals (kPa) (15 pounds per square inch (psi)); system pressure: (2068.4 kPa) 300psi to (3447.4 kPa) 500 psi; no air heat.

### FLUID ACQUISITION TIME

Test constructions were made and the time needed to absorb 100 milliliters (ml) of a saline solution (0.09% saline, with blue dye) was measured.

The construction consisted of a core consisting of 10 layers of core material (32 gsm cellulose/tissue composite) and a top sheet (15 gsm spun bond polypropylene nonwoven). The acquisition distribution layer to be tested was layered between the core and the top sheet.

A test fixture including a 2.5 centimeter (cm) wide (internal diameter) tube, 24 cm long open on both ends and fixed to a metal base fitted with a similar sized opening, was placed with the metal base side down on the top sheet side of the diaper construction. A 250 ml capacity separatory funnel (set to give a flow of 7 ml/second when valve open) was placed into the tube, with the valve locked. The saline solution poured into the funnel. A timer was started as the valve was released. The timer was stopped when the last amount of fluid had been absorbed into the top sheet.

**Table 1**

| | Comparative A | Composition A |
|---|---|---|
| ATEVA 1850A | 100 | |
| ATEVA 2850A | | 100 |
| Brookfield Viscosity at 148.9°C (cP) | | 28300 |
| Brookfield Viscosity at 162.8°C (cP) | | 18300 |
| Brookfield Viscosity at 177.8°C (cP) | 87000 | 12250 |
| Brookfield Viscosity at 190°C (cP) | | |
| Application Temperature °F (°C) | 350 (178) | 350 (178) |
| Spray Test Method Observations | Material would not spray; viscosity too high | Material formed nice web of fibers on top of the nonwoven substrate |

**Table 2**

| | Control 1 (NO ADL layer) | Control 2 (Standard ADL) | Example 1 | Example 2 |
|---|---|---|---|---|
| **Composite Construction (top to bottom)** | | | | |
| Top sheet | yes | yes | yes | yes |
| Standard ADL (80 gsm) | | yes | | |
| Self-supporting fiberized web consisting of Composition A (40 gsm) | | | yes | |
| Self-supporting fiberized web consisting of Composition A (80 gsm) | | | | yes |
| Core (10 layers of air laid) | yes | yes | yes | yes |
| | | | | |
| Fluid Acquisition Time (seconds) | 67.8 | 25.4 | 46.6 | 41.2 |

| | | | | |
|---|---|---|---|---|
| Standard ADL is a 80 gsm basis weight nonwoven (7.5 cm by 17 cm) obtained from Tecnosur WINNY brand diaper (Size 0, newborn) | | | | |

**Table 3**

| | | Example 3 | Example 4 | Example 5 |
|---|---|---|---|---|
| | | Weight % | Weight % | Weight % |
| ATEVA 2842 | | 70 | | |
| AFFINITY GA 1900 | | | 70 | 60 |
| AFFINITY GA1950 | | | | 10 |
| SARAWAX SX105 | | 30 | 30 | 30 |
| | | | | |
| Brookfield Viscosity at 148.9°C (300°F) (cP) | | 10180 | 3825 | 4638 |
| Brookfield Viscosity at 162.8°C (325°F) (cP) | | 7025 | 2745 | 3338 |
| Brookfield Viscosity at 176.7°C (350°F) (cP) | | 5025 | 2035 | 2475 |
| | Basis Weight | | | |
| Fluid Acquisition Time (seconds) Formed at 148.9°C (300°F) | 80 | | | 29 |
| Fluid Acquisition Time (seconds) Formed at 162.8°C (325°F) | 80 | | 25 | |
| Fluid Acquisition Time (seconds) Formed at 162.8°C (325°F) | 60 | | 39 | |
| Fluid Acquisition Time (seconds) Formed at 176.7°C (350°F) | 80 | 22 | 26 | 22 |
| Fluid Acquisition Time (seconds) Formed at 176.7°C (350°F) | 60 | 33 | 39 | 30 |

Other embodiments are within the claims.

## Claims

1. A disposable absorbent article comprising:
a top sheet;
an absorbent core; and
a self-supporting fiberized web situated between the top sheet and the absorbent core,
wherein the self-supporting fiberized web comprises a hot melt composition having a viscosity of no greater than 50,000 cP at 178°C, as measured by the viscosity test method described herein, and the self-supporting fiberized web has a basis weight of about 10 to about 200 grams per square meter and is free of absorbent materials.

2. The disposable absorbent article of claim 1, further comprising a standard acquisition distribution layer situated either directly above or directly below the self-supporting fiberized web.

3. The disposable absorbent article of claim 1, wherein the hot melt composition is tack free at room temperature.

4. The disposable absorbent article of claim 1, wherein the hot melt composition has a viscosity of from about 100 cP to about 30,000 cP at 178°C, as measured by the viscosity test method described herein.

5. The disposable absorbent article of claim 1, wherein the hot melt composition comprises an ethylene copolymer.

6. The disposable absorbent article of claim 5, wherein an ethylene copolymer is selected from a group consisting of ethylene olefin copolymers and ethylene vinyl acetate copolymers.

7. The disposable absorbent article of claim 1, wherein the hot melt composition comprises an ethylene copolymer and a wax having a Ring and Ball Softening Point of from about 95°C to about 140°C.

8. The disposable absorbent article of claim 7, wherein the wax is selected from a group consisting of Fischer Tropsch wax and polyethylene wax.

9. The disposable absorbent article of claim 1, wherein the hot melt composition comprises from about 50 % by weight to about 100 % of a thermoplastic polymer.

10. A method of forming a self-supporting fiberized web being free of absorbent materials, the method comprising the steps of:
a. obtaining a hot melt composition having a viscosity of no greater than 50,000 cP at 178°C, as measured by the viscosity test method described herein;
b. heating the hot melt composition until molten;
c. spraying the hot melt composition, through an applicator;
d. mixing the hot melt composition with air as it comes through the orifices; and
e. applying the hot melt composition as a self-supporting fiberized web having a basis weight of about 10 to about 200 grams per square meter onto a substrate selected from a group consisting of a top sheet, an absorbent core, a core wrap and a standard acquisition distribution layer,
wherein the hot melt composition is cooled such that it forms a layer on top of the substrate rather than penetrating into the substrate as it makes contact with the substrate.

11. The method of claim 10, wherein the applicator comprises from 1 to 30 orifices/2.54 cm (1 to 30 orifices/inch).

12. The method of claim 10, wherein the air is not heated.

13. The method of claim 10, wherein the air is cooled.

## Patentansprüche

1. Ein absorbierender Wegwerfartikel, der Folgendes umfasst:
ein Deckblatt;
einen saugfähigen Kern; und
eine selbsttragende faserige Bahn, die sich zwischen dem Deckblatt und dem absorbierenden Kern befindet,
wobei die selbsttragende faserige Bahn eine Heißschmelzzusammensetzung mit einer Viskosität von nicht mehr als 50.000 cP bei 178°C, gemessen mit dem hierin beschriebenen Viskositätstestverfahren, umfasst und die selbsttragende faserige Bahn ein Flächengewicht von etwa 10 bis etwa 200 Gramm pro Quadratmeter aufweist und frei von absorbierenden Materialien ist.

2. Der absorbierende Wegwerfartikel gemäß Anspruch 1, weiterhin umfassend eine Standard-Erwerbsverteilungsschicht, die sich entweder direkt über oder direkt unter der selbsttragenden faserige Bahn befindet.

3. Der absorbierende Wegwerfartikel gemäß Anspruch 1, wobei die Heißschmelzzusammensetzung bei Raumtemperatur klebfrei ist.

4. Der absorbierende Wegwerfartikel gemäß Anspruch 1, wobei die Heißschmelzzusammensetzung eine Viskosität von etwa 100 cP bis etwa 30.000 cP bei 178°C aufweist, gemessen mit dem hierin beschriebenen Viskositätstestverfahren.

5. Der absorbierende Wegwerfartikel gemäß Anspruch 1, wobei die Heißschmelzzusammensetzung ein Ethylen-Copolymer umfasst.

6. Der absorbierende Wegwerfartikel gemäß Anspruch 5, wobei ein Ethylen-Copolymer aus einer Gruppe ausgewählt ist, die aus Ethylen-Olefin-Copolymeren und EthylenVinylacetat-Copolymeren besteht.

7. Der absorbierende Wegwerfartikel gemäß Anspruch 1, wobei die Heißschmelzzusammensetzung ein Ethylen-Copolymer und ein Wachs mit einem Ring- und Kugel-Erweichungspunkt von etwa 95°C bis etwa 140°C umfasst.

8. Der absorbierende Wegwerfartikel gemäß Anspruch 7, wobei das Wachs aus einer Gruppe ausgewählt ist, die aus Fischer-Tropsch-Wachs und Polyethylenwachs besteht.

9. Der absorbierende Wegwerfartikel gemäß Anspruch 1, wobei die Heißschmelzzusammensetzung etwa 50 Gew.-% bis etwa 100 Gew.-% eines thermoplastischen Polymers umfasst.

10. Ein Verfahren zur Herstellung einer selbsttragenden faserigen Bahn, die frei von absorbierenden Materialien ist, wobei das Verfahren die folgenden Schritte umfasst:
a. Erhalten einer Heißschmelzzusammensetzung mit einer Viskosität von nicht mehr als 50.000 cP bei 178°C, gemessen mit dem hier beschriebenen Viskositätstestverfahren;
b. Erhitzen der Heißschmelzzusammensetzung, bis sie geschmolzen ist;
c. Sprühen der Heißschmelzzusammensetzung durch einen Applikator;
d. Mischen der Heißschmelzzusammensetzung mit Luft, wenn sie durch die Öffnungen kommt; und
e. Aufbringen der Heißschmelzzusammensetzung als selbsttragende faserige Bahn mit einem Flächengewicht von etwa 10 bis etwa 200 Gramm pro Quadratmeter auf ein Substrat, das aus einer Gruppe ausgewählt ist, die aus einer Decklage, einem absorbierenden Kern, einer Kernumhüllung und einer Standard-Erwerbsverteilungsschicht besteht,
wobei die Schmelzklebstoffzusammensetzung so abgekühlt wird, dass sie eine Schicht auf dem Substrat bildet und nicht in das Substrat eindringt, wenn sie mit dem Substrat in Kontakt kommt.

11. Das Verfahren gemäß Anspruch 10, wobei der Applikator 1 bis 30 Öffnungen/2,54 cm (1 bis 30 Öffnungen/Zoll) aufweist.

12. Das Verfahren gemäß Anspruch 10, wobei die Luft nicht erhitzt wird.

13. Das Verfahren gemäß Anspruch 10, wobei die Luft gekühlt wird.

## Revendications

1. Article absorbant jetable comportant :
une feuille supérieure,
un noyau absorbant, et
une bande défibrée auto-portante située entre la feuille supérieure et le noyau absorbant,
dans lequel la bande défibrée auto-portante comporte une composition thermofusible ayant une viscosité non supérieure à 50 000 cP à 178 °C lorsqu'elle est mesurée par la méthode d'essai de viscosité décrite dans la description, et la bande défibrée auto-portante a un grammage d'environ 10 jusqu'à environ 200 grammes par mètre carré et est exempte de matières absorbantes.

2. Article absorbant jetable selon la revendication 1, comportant en outre une couche de distribution d'acquisition standard située directement au-dessus ou directement au-dessous de la bande défibrée auto-portante.

3. Article absorbant jetable selon la revendication 1, dans lequel la composition thermofusible est non collante à température ambiante.

4. Article absorbant jetable selon la revendication 1, dans lequel la composition thermofusible a une viscosité d'environ 100 cP à environ 30 000 cP à 178 °C, lorsqu'elle est mesurée par la méthode d'essai de viscosité décrite dans la description.

5. Article absorbant jetable selon la revendication 1, dans lequel la composition thermofusible est un copolymère d'éthylène.

6. Article absorbant jetable selon la revendication 5, dans lequel un copolymère d'éthylène est choisi parmi un groupe constitué de copolymères d'éthylène et d'oléfine et de copolymères d'éthylène et d'acétate de vinyle.

7. Article absorbant jetable selon la revendication 1, dans lequel la composition thermofusible comporte un copolymère d'éthylène et une cire ayant un point de ramollissement par anneau et bille d'environ 95 °C jusqu'à environ 140 °C.

8. Article absorbant jetable selon la revendication 7, dans lequel la cire est choisie parmi un groupe constitué de cire de Fischer Tropsch et de cire de polyéthylène.

9. Article absorbant jetable selon la revendication 1, dans lequel la composition thermofusible comporte environ 50 % en poids jusqu'à à environ 100 % d'un polymère thermoplastique.

10. Procédé de formation d'une bande défibrée auto-portante étant exempte de matières absorbantes, le procédé comportant les étapes consistant à :
a. obtenir une composition thermofusible ayant une viscosité non supérieure à 50 000 cP à 178 °C, lorsqu'elle est mesurée par la méthode d'essai de viscosité décrite dans la description,
b. chauffer la composition thermofusible jusqu'à ce qu'elle soit fondue,
c. pulvériser la composition thermofusible, à travers un applicateur,
d. mélanger la composition thermofusible avec de l'air lorsqu'elle arrive à travers les orifices, et
e. appliquer la composition thermofusible sous la forme d'une bande défibrée auto-portante ayant un grammage d'environ 10 à environ 200 grammes par mètre carré sur un substrat choisi parmi un groupe constitué d'une feuille supérieure, d'un noyau absorbant, d'une enveloppe de noyau et d'une couche de distribution d'acquisition standard,
dans lequel la composition thermofusible est refroidie de telle sorte qu'elle forme une couche au-dessus du substrat plutôt que de pénétrer dans le substrat lorsqu'elle fait contact avec le substrat.

11. Procédé selon la revendication 10, dans lequel l'applicateur comporte de 1 à 30 orifices/2,54 cm (1 à 30 orifices/pouce).

12. Procédé selon la revendication 10, dans lequel l'air n'est pas chauffé.

13. Procédé selon la revendication 10, dans lequel l'air est refroidi.
